# EUROPÄISCHE PATENTSCHRIFT

(11) **EP 3 291 782 B1**
(45) Veröffentlichungstag und Bekanntmachung des Hinweises auf die Patenterteilung: **06.03.2019**
(21) Anmeldenummer: 16722843.6
(22) Anmeldetag: 04.05.2016
(51) Int. Cl.: A61F 13/08

(54) **KOMPRESSIONSARTIKEL**
COMPRESSION DEVICE
ARTICLE DE CONTENTION

(30) Priorität: 04.05.2015 DE 102015106903
(43) Veröffentlichungstag der Anmeldung: 14.03.2018
(73) Patentinhaber: BSN -Jobst GmbH, 46446 Emmerich (DE)
(72) Erfinder: TÜRK, Alexander, 42279 Wuppertal (DE); CHROST, Marius, 42107 Wuppertal (DE); BAUER, Joachim, 22761 Hamburg (DE); GREVE, Jürgen, 46446 Emmerich (DE)
(74) Vertreter: Kohler Schmid Möbus Patentanwälte
(86) Internationale Anmeldenummer: PCT/EP2016/060085
(87) Internationale Veröffentlichungsnummer: WO 2016/177829

(56) Entgegenhaltungen:
- FR-A- 1 519 361
- US-A- 3 800 331
- US-A1- 2012 324 961

## Beschreibung

Die Erfindung betrifft einen Kompressionsartikel, der aus einem elastischen textilen Material, insbesondere einem Gestrick hergestellt und mit zumindest einem elastischen Halteband zur Rutschsicherung auf dem Körperteil versehen ist.

Solche Kompressionsartikel sind insbesondere in der Form von Bein- oder Kniestrümpfen bereits bekannt. So beschreibt beispielsweise das Gebrauchsmuster DE 82 17 651 U1 einen medizinischen Gummistrumpf mit einem Halteband, das als Häkelgalonband ausgeführt ist und auf seiner Innenseite mit einer Vielzahl von Noppen aus Silikon versehen ist.

Die Noppen auf der Innenseite des Haltebandes verhindern zuverlässig ein Herunterrutschen des Strumpfes, schränken jedoch gleichzeitig die Atmungsaktivität der Haut ein. Die Noppen oder Streifen der bekannten Haltebänder sind dabei relativ großflächig und liegen bei medizinischen Kompressionsartikeln, die täglich bis zu sechzehn Stunden zu tragen sind, immer an den gleichen Hautstellen auf. Es kann daher zu Hautreizungen kommen, die den Tragekomfort der Artikel einschränken.

Außerdem ist die Herstellung der bekannten Haltebänder durch das nachträgliche Aufbringen der Silikonnoppen oder -streifen relativ aufwändig und kostenintensiv.

Die US 3 800 331 A beschreibt einen medizinischen Strumpf mit einem Halteband, das aus einem Gewebe mit Schussfäden mit einem hohen Reibungskoeffizienten hergestellt ist. Die Schussfäden liegen auf einer Gesamtfläche von weniger als 50 % frei auf der Innenseite des Haltebandes. Ein ähnlicher Strumpf ist auch in der US 2012/0324961 A1 offenbart. Dort ist das Halteband unter Verwendung von an der Innenseite freiliegenden Garnen mit einem hohen Reibungskoeffizienten gestrickt. Auch bei diesen bekannten Haltebändern kann es zu relativ großflächigen Hautreizungen kommen.

Der vorliegenden Erfindung liegt daher die Aufgabe zugrunde, einen Kompressionsartikel vorzuschlagen, der die genannten Nachteile vermeidet.

Die Aufgabe wird gelöst durch einen Kompressionsartikel mit den Merkmalen des Patentanspruchs 1. Das elastische Gestrick, Gewirk oder Gewebe kann in der Form dem zu stützenden Körperteil anatomisch angepasst sein. Das Halteband kann vorzugsweise ein gewebtes Band sein, das unter Verwendung eines Friktionsfadens hergestellt ist, der an der Bandinnenseite stellenweise an der Oberfläche es Bandes liegt. Auch gestrickte oder raschelgewirkte Bänder sind denkbar. Auf der Bandaußenseite kann der Friktionsfaden vollständig von anderen Fäden abgedeckt sein.

Durch das Einarbeiten der Friktionsfäden in das Band ist ein nachträgliches Auftragen von Silikonnoppen oder Streifen überflüssig. Darüber hinaus weisen die einzelnen Stellen, an der der Friktionsfaden an der Oberfläche des Haltebandes liegt, nur äußerst geringe Abmessungen auf, sodass die Atmungsaktivität der Haut nur wenig beeinträchtigt wird. Die verwendeten Fadenstärken betragen üblicherweise zwischen 15 dtex und 5.000 dtex. Der Tragekomfort der erfindungsgemäßen Kompressionsartikel ist daher äußerst hoch. Dennoch übt das Halteband eine vergleichbare Haltefunktion aus wie die Haltebänder bekannter Kompressionsartikel. Als Friktionsfäden kommen alle Garne mit einem Friktionskoeffizienten von größer als 0,4 in Betracht.

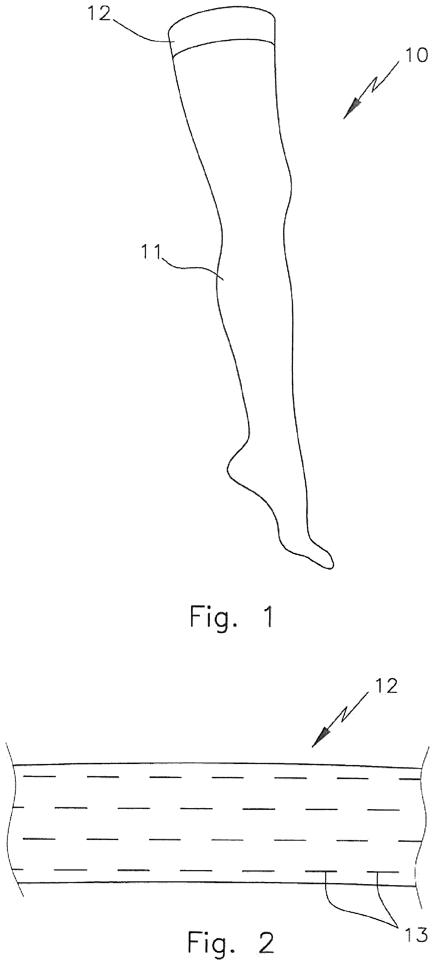

Der Friktionskoeffizient wird dabei nach dem im ASTM Standard D 3108-95 beschriebenen Verfahren mit folgenden Erweiterungen bestimmt: Es wird ein Gerät gemäß Fig. 2 dieses Standards verwendet und der zu untersuchende Faden in einem Winkel von 163,5° um einen Keramikstab herumgeführt, wobei der Stab einen Durchmesser von 8 mm aufweist. Die Vorspannung, mit dem der zu untersuchende Faden beaufschlagt wird, beträgt 3,0 g, unabhängig vom dtex-Wert des untersuchten Fadens. Der Wert des Friktionskoeffizienten wird dann aus den Messwerten für die Eingangsspannung und die Ausgangsspannung wie im Standard beschrieben berechnet.

Die Gesamtfläche der Stellen, an denen der Friktionsfaden an der Oberfläche der Bandinnenseite liegt beträgt, weniger als 25 % der Oberfläche, bevorzugt weniger als 15 % der Bandinnenseite. Die Haltefunktion des Haltebandes wird dadurch nicht beeinträchtigt. Dies ist deutlich weniger Silikonfläche als bei herkömmlichen Haltebändern mit aufgebrachten Silikonnoppen oder Streifen. Hautreizungen treten bei solch geringen, wenig atmungsaktiven Flächen nur in geringem Maß auf.

Vorzugsweise kann das Halteband einen Kompressionsdruck ausüben, der in einem festen Verhältnis zum Kompressionsdruck des Gestricks steht, damit der gewünschte Kompressionsdruck auch an den Abschlussrändern des Kompressionsartikels gewährleistet ist. Das Gestrick weist einen kontinuierlichen Abfall des Kompressionsdrucks von distal nach proximal auf. Das Halteband kann diesen Druckabfall fortsetzen oder auch einen höheren Druck als das benachbarte Gestrick ausüben, um die Haltefunktion zu verbessern.

Weitere Vorteile ergeben sich, wenn die Stellen, an denen der Friktionsfaden an der Oberfläche der Bandinnenseite liegt, gleichmäßig über die Bandinnenseite verteilt sind. Die Haltefunktion ist dann an allen Stellen des Haltebandes die gleiche. Außerdem wird die Haut gleichmäßig belastet. Selbstverständlich ist jedoch auch eine ungleichmäßige Verteilung der Stellen, an denen die Friktionsfäden an der Oberfläche des Bandes zu liegen kommen, möglich, sofern dies beispielsweise aufgrund besonders empfindlicher Hautzonen oder dergleichen erforderlich sein sollte.

Bei einer bevorzugten Variante des Kompressionsartikels besteht ein Teil der Kettfäden des Haltebandes aus dem Friktionsfaden. Auf der Außenseite des Haltebandes können diese Kettfäden vollständig und auf der Innenseite teilweise von Schussfäden aus einem anderen Material überdeckt sein.

Als Friktionsfadenmaterial kommen Spandex, natürliches Gummi, Silikon oder synthetisches Gummi in Frage. Es ist außerdem möglich, beschichtete Garne zu verwenden, wobei die Beschichtung aus vulkanisierten Elastomeren, flüssigem Silikon, Silikongummi und/oder Polyurethan-Elastomeren bestehen kann.

Das Halteband kann neben dem Friktionsfaden vorzugsweise umsponnene elastische Fäden und Polyamidfäden enthalten. Mit den elastischen Fäden lässt sich der gewünschte Kompressionsdruck einstellen, während die Polyamidfäden für die erforderliche Stabilität des Haltebandes sorgen.

Der Friktionsfaden kann vorzugsweise transparent oder transluzent sein und dadurch optisch nicht auffallen. Er lässt sich dadurch auch mit jeder Farbe der anderen Fäden problemlos kombinieren.

Weiter kann das Halteband in an sich bekannter Weise an das Gestrick angenäht sein. Auch eine Schweißnaht zwischen dem Gestrick und dem Halteband ist prinzipiell möglich.

Die Zeichnung zeigt ein mögliches Ausführungsbeispiel eines erfindungsgemäßen Kompressionsartikels.

Es zeigen:
- Fig. 1: eine schematische Darstellung eines Kompressionsstrumpfes mit einem Halteband;
- Fig. 2: eine Detaildarstellung der Innenseite des Haltebandes aus Fig. 1.

Der Kompressionsstrumpf 10 aus Fig. 1 ist ein Beinstrumpf, der sich bis zum Oberschenkel des Trägers erstreckt. Er besteht aus einem der anatomischen Form des Beins angepassten Gestrick 11, an dessen oberem Rand ein Halteband 12 angenäht ist. Das Halteband 12 sichert den Strumpf 10 gegen Rutschen.

Wie die Detailansicht der Innenseite des Bandes 12 aus Fig. 2 zeigt, handelt es sich bei dem Halteband 12 um ein gewebtes Band, das unter Verwendung eines Friktionsfadens 13 hergestellt ist, der an gleichmäßig über die Oberfläche der Bandinnenseite verteilten Stellen an der Oberfläche der Bandinnenseite liegt und somit mit der Haut des Trägers des Kompressionsartikels 10 in Kontakt kommt. Der Friktionsfaden 13 sichert damit die Haltefunktion des Haltebandes 12. Dabei schränkt er die Atmungsaktivität des Haltebandes 12 praktisch nicht ein, sodass der Kompressionsstrumpf einen hohen Tragekomfort aufweist.

## Patentansprüche

1. Kompressionsartikel, der aus einem elastischen textilen Material, insbesondere einem Gestrick (11) hergestellt ist und der mit zumindest einem elastischen Halteband (12) als Rutschsicherung auf dem Körperteil versehen ist, wobei das Halteband (12) unter Verwendung eines Friktionsfadens (13) hergestellt ist, der an der Bandinnenseite stellenweise an der Oberfläche des Bandes (12) liegt, **dadurch gekennzeichnet, dass** die Gesamtfläche der Stellen, an denen der Friktionsfaden (13) an der Oberfläche der Bandinnenseite liegt, weniger als 25 %, bevorzugt weniger als 15 % der Oberfläche der Bandinnenseite beträgt.

2. Kompressionsartikel nach Anspruch 1, **dadurch gekennzeichnet, dass** das Halteband (12) ein gewebtes Band ist.

3. Kompressionsartikel nach Anspruch 1 oder 2, **dadurch gekennzeichnet, dass** der Friktionsfaden (13) auf der Bandaußenseite vollständig von anderen Fäden abgedeckt ist.

4. Kompressionsartikel nach einem der vorhergehenden Ansprüche, **dadurch gekennzeichnet, dass** das Halteband (12) einen Kompressionsdruck ausübt, der in einem festen Verhältnis zum Kompressionsdruck des angrenzenden Bereichs des Gestricks (11) steht, sodass das Halteband (12) den kontinuierlichen Abfall des Kompressionsdrucks von distal nach proximal des Gestricks (11) fortsetzt oder einen höheren Druck als das benachbarte Gestrick (11) ausübt.

5. Kompressionsartikel nach einem der vorhergehenden Ansprüche, **dadurch gekennzeichnet, dass** das Gestrick (11) in der Form dem zu stützenden Körperteil anatomisch angepasst ist.

6. Kompressionsartikel nach einem der vorhergehenden Ansprüche, **dadurch gekennzeichnet, dass** die Stellen, an denen der Friktionsfaden (13) an der Oberfläche der Bandinnenseite liegt, gleichmäßig über die Bandinnenseite verteilt sind.

7. Kompressionsartikel nach einem der vorhergehenden Ansprüche 2 bis 6, **dadurch gekennzeichnet, dass** ein Teil der Kettfäden des Haltebandes (12) aus dem Friktionsfaden (13) besteht.

8. Kompressionsartikel nach einem der vorhergehenden Ansprüche, **dadurch gekennzeichnet, dass** das Halteband (12) außerdem umsponnene elastische Fäden und Polyamidfäden enthält.

9. Kompressionsartikel nach einem der vorhergehenden Ansprüche, **dadurch gekennzeichnet, dass** der Friktionsfaden (13) transparent oder transluzent ist.

10. Kompressionsartikel nach einem der vorhergehenden Ansprüche, **dadurch gekennzeichnet, dass** das Halteband (12) an das Gestrick (11) angenäht ist.

## Claims

1. Compression article produced from an elastic textile material, in particular a knitted fabric (11), and provided with at least one elastic retaining band (12) as an anti-slip means on the body part, wherein the retaining band (12) is produced using a friction thread (13) situated at some points on the surface of the band (12) on the inner face of the band, **characterised in that** the total area of the points at which the friction thread (13) is situated on the surface of the inner face of the band is less than 25 %, preferably less than 15 %, of the surface of the inner face of the band.

2. Compression article according to claim 1, **characterised in that** the retaining band (12) is a woven band.

3. Compression article according to claim 1 or claim 2, **characterised in that** the friction thread (13) is completely covered by other threads on the outer face of the band.

4. Compression article according to one of the preceding claims, **characterised in that** the retaining band (12) exerts compression in a fixed ratio to the compression of the adjoining region of the knitted fabric (11) so that the retaining band (12) continues the continuous reduction in compression from the distal to the proximal end of the knitted fabric (11) or exerts greater pressure than the adjacent knitted fabric (11).

5. Compression article according to one of the preceding claims, **characterised in that** the knitted fabric (11) is adapted anatomically in shape to the part of the body to be supported.

6. Compression article according to one of the preceding claims, **characterised in that** the points at which the friction thread (13) is situated on the surface of the inner face of the band are distributed in a uniform manner over the inner face of the band.

7. Compression article according to one of the preceding claims 2 to 6, **characterised in that** some of the warp threads of the retaining band (12) consist of the friction thread (13).

8. Compression article according to one of the preceding claims, **characterised in that** the retaining band (12) moreover contains core-spun elastic threads and polyamide threads.

9. Compression article according to one of the preceding claims, **characterised in that** the friction thread (13) is transparent or translucent.

10. Compression article according to one of the preceding claims, **characterised in that** the retaining band (12) is sewn on to the knitted fabric (11).

## Revendications

1. Article de contention, qui est fabriqué à partir d'une matière textile élastique, en particulier d'un tricot (11), et qui est muni d'au moins une bande de maintien élastique (12) comme protection contre le glissement sur la partie corporelle, dans lequel la bande de maintien (12) est fabriquée en utilisant un fil de friction (13) qui, du côté interne de bande, se trouve par endroits au niveau de la surface de la bande (12), **caractérisé en ce que** la surface totale des endroits où le fil de friction (13) se trouve au niveau de la surface du côté interne de bande est égale à moins de 25 %, de préférence moins de 15 %, de la surface du côté interne de bande.

2. Article de contention selon la revendication 1, **caractérisé en ce que** la bande de maintien (12) est une bande tissée.

3. Article de contention selon la revendication 1 ou 2, **caractérisé en ce que** le fil de friction (13) est complètement recouvert par d'autres fils sur le côté externe de bande.

4. Article de contention selon l'une quelconque des revendications précédentes, **caractérisé en ce que** la bande de maintien (12) exerce une pression de contention qui est dans un rapport fixe à la pression de contention de la zone adjacente du tricot (11) de telle sorte que la bande de maintien (12) poursuit la diminution continue de la pression de contention de la zone distale à la zone proximale du tricot (11) ou exerce une pression plus grande que le tricot (11) voisin.

5. Article de contention selon l'une quelconque des revendications précédentes, **caractérisé en ce que** le tricot (11) est adapté anatomiquement par sa forme à la partie corporelle à soutenir.

6. Article de contention selon l'une quelconque des revendications précédentes, **caractérisé en ce que** les endroits où le fil de friction (13) se trouve au niveau de la surface du côté interne de bande sont répartis régulièrement sur tout le côté interne de bande.

7. Article de contention selon l'une quelconque des revendications 2 à 6, **caractérisé en ce qu'**une partie des fils de chaîne de la bande de maintien (12) est constituée du fil de friction (13).

8. Article de contention selon l'une quelconque des revendications précédentes, **caractérisé en ce que** la bande de maintien (12) contient en outre des fils élastiques guipés et des fils de polyamide.

9. Article de contention selon l'une quelconque des revendications précédentes, **caractérisé en ce que** le fil de friction (13) est transparent ou translucide.

10. Article de contention selon l'une quelconque des revendications précédentes, **caractérisé en ce que** la bande de maintien (12) est cousue au tricot (11).
